# FASCICULE DE BREVET EUROPEEN

(11) **EP 0 623 145 B1**
(45) Date de publication et mention de la délivrance du brevet: **25.06.1997**
(21) Numéro de dépôt: 93914478.8
(22) Date de dépôt: 08.01.1993
(51) Int. Cl.: C07K 7/06, C12P 21/08, G01N 33/04

(54) **PEPTIDES DU CASEINOMACROPEPTIDE, ANTICORPS CONTRE LESDITS PEPTIDES ET UTILISATIONS**
PEPTIDE VON DEM CASEINOMACROPEPTID, ANTIKORPER DAGEGEN UND IHRE ANDWENDUNGEN
PEPTIDES OF THE CASEIN MACROPEPTIDE, ANTIBODIES DIRECTED AGAINST SAID PEPTIDES, AND APPLICATIONS

(30) Priorité: 10.01.1992 FR 9200188
(43) Date de publication de la demande: 09.11.1994
(73) Titulaire: SYSTEMS BIO-INDUSTRIES, F-75008 Paris (FR); SANOFI DIAGNOSTICS PASTEUR, 92430 Marnes La Coquette (FR)
(72) Inventeur: COLLIN, Jean-Claude, F-29800 Poligny (FR); PERROD, Jean-Louis, F-01380 Bage-le-Chatel (FR)
(74) Mandataire: Ores, Irène
(86) Numéro de dépôt international: FR9300014
(87) Numéro de publication internationale: WO9314117

(56) Documents cités:
- EP-A- 0 358 938
- EP-A- 0 397 571
- The Biochemical Journal, vol. 271, no. 1, 1 octobre 1990, London, GB, J. LEONIL et al., pages 247-252
- Biochimica et Biophysica Acta, vol. 995, no. 3, 1989, Amsterdam, NL, H.B. DROHSE et al., pages 221-224
- Chemical Abstracts, vol. 105, no. 13, 29 septembre 1986, Columbus, Ohio, US, H.A. MEHRENS et al., page 560
- Biological Abstracts, vol. 91, no. 7, 1991, Philadelphia, PA, US, H. OTANI et al., page AB390

## Description

L'Invention est relative à un procédé permettant d'évaluer le degré de dénaturation protéolytique du lait et des produits laitiers.

Les phénomènes de protéolyse et, en particulier, ceux dus aux protéases bactériennes sont responsables d'une baisse de qualité des produits laitiers.

Les bactéries responsables de ces phénomènes sont essentiellement des bactéries Gram négatif, et surtout des *Pseudomonas.* Il s'agit de bactéries psychrotrophes capables de se développer à basse température ; le stockage du lait à 4°C favorise le développement de ces bactéries, aux dépens d'autres espèces.

L'action des protéases des bactéries psychrotrophes a été étudiée essentiellement au niveau de la caséine. Il semble que la fraction la plus rapidement dégradée soit la caséine κ ; la caséine β est également attaquée de façon significative, et à un degré moindre, la caséine asl est également dégradée. La protéolyse de la caséine κ par les protéases des bactéries psychrotrophes libère un peptide d'environ une soixantaine d'acides aminés (par exemple, 64 acides aminés pour le lait de vache), le caséino-macropeptide (CMP), également dénommé glycomacropeptide (GMP), et correspondant à l'extrémité C-terminale de la caséine κ.

Il a été proposé (Demande EP 0 397 571) d'utiliser le CMP pour la prévention et le traitement des thromboses.

L'activité protéasique des bactéries psychrotrophes est thermorésistante, et résiste aux traitements de stérilisation UHT. La persistance de cette activité dans les produits UHT, diminue le temps de conservation de ces produits, et leurs qualités organoleptiques. Il est donc souhaitable de disposer d'un test permettant d'évaluer la protéolyse (ou le potentiel protéolytique) due aux bactéries psychrotrophes dans le lait et les produits laitiers.

Un tel test, pratiqué sur le lait à son arrivée à la laiterie, permettrait de déterminer les traitements et les transformations les mieux adaptés à la qualité du lait reçu. Il serait ainsi possible d'éviter certains accidents de fabrication (gélification des laits UHT, flaveurs amères, rancissement, baisse des rendements fromagers).

D'autre part, un tel test serait également très utile à la fabrication fromagère. En effet, les enzymes protéolytiques des bactéries psychrotrophes jouent un rôle certain dans la maturation des fromages. La détermination de l'activité protéolytique potentielle avant la fabrication permettrait de mieux contrôler la maturation en modulant les conditions d'affinage.

Plusieurs techniques ont, en conséquence, été proposées dans le but d'évaluer l'activité protéolytique dans le lait, et en particulier celle due aux bactéries psychrotrophes.

Il a par exemple, été proposé d'évaluer directement la flore psychrotrophe, responsable de la protéolyse. Cette méthode qui fait intervenir des techniques de culture et de numération des bactéries présente tous les inconvénients y afférant il faut disposer d'un équipement spécifique relativement important et d'un personnel spécialisé et, d'autre part, les résultats ne sont obtenus qu'après le délai nécessaire à la croissance des cultures bactériennes. Récemment, des techniques rapides indirectes (bioluminescence, impédancemétrie, etc...), ont été proposées. Toutefois, elles nécessitent un appareilage spécifique, sont plus ou moins adaptées à des cultures hétérogènes comme le lait et les produits laitiers, et sont en pratique peu utilisées dans l'industrie laitière.

Il a également été proposé de doser directement ces protéases ; ces dosages sont effectués soit sur la base de l'activité enzymatique, soit directement par quantification immunochimique des protéases.

Le dosage de l'activité enzymatique s'effectue par exemple en déterminant l'hydrolyse de substrats naturels ou artificiels, libérant des produits marqués (colorés, fluorescents, ou radioactifs) . A titre d'exemple, on citera la technique HPA [ CLIFFE et LAW, J. Dairy Sci., 49, 209-219 (1982)], dans laquelle le substrat est du collagène dénaturé par liaison covalente avec un colorant bleu. Cette technique permet de détecter les protéases produites par environ 2.10⁶ psychrotrophes/ml.

Des méthodes immunochimiques ont été proposées, par exemple, pour la protéinase P1 de *Pseudomonas fluorescens* [BRIKELAND et al., Appl. Environ. Microbiol., 49, 382-387]. Le dosage de cette protéinase par la technique ELISA permet de détecter l'équivalent d'environ 10⁷ psychrotrophes/ml.

D'autres techniques font appel à la mesure des acides aminés ou de l'azote non protéique libérés par la protéolyse. Ces méthodes sont toutefois, malaisées à mettre en oeuvre, car elles demandent une séparation préalable des produits de l'hydrolyse. Elles permettent de détecter environ 5.10⁶ psychrotrophes/ml.

Enfin, d'autres méthodes sont basées sur la détection des produits de l'hydrolyse des caséines. Pour la caséine κ, il a été proposé de doser les deux produits de dégradation qui sont la paracaséine κ et le caséinomacropeptide (CMP).

Actuellement, la paracaséine κ n'est décelable que par électrophorèse, ce qui représente une méthode relativement lourde.

En ce qui concerne le CMP, qui est un glycopeptide, il a été proposé de le doser par mesure de l'acide sialique, de l'acide N-acétyl-neuraminique, ou par chromatographie sur DEAE cellulose. Il s'agit toutefois de techniques lourdes, et les résultats obtenus sont peu précis.

Afin de disposer d'une méthode alliant rapidité, simplicité de mise en oeuvre et fiabilité, les Inventeurs ont entrepris la mise au point d'une technique immunologique de dosage du CMP.

Ils ont dans un premier temps obtenu des anticorps anti-CMP [COLLIN et al. Communication au XXème Congrès International de Laiterie, Montréal, 8 - 12 Octobre 1990], dirigés contre le CMP obtenu par hydrolyse de la caséine κ, et ont utilisé ces anticorps dans le but d'évaluer par ELISA la protéolyse de la caséine κ dans le lait.

Toutefois, la préparation du CMP par hydrolyse de la caséine κ nécessite des opérations de purification également longues et coûteuses pour éliminer toute contamination par la caséine κ non hydrolysée. En outre, les Inventeurs ont constaté que, même lorsque l'on utilise comme immunogène une préparation de CMP parfaitement purifiée, non contaminée par la caséine κ, on obtient malgré tout des anticorps présentant des réactions croisées avec la caséine κ non hydrolysée ; ces réactions ne sont pas éliminées même après plusieurs chromatographies d'affinité.

LEONIL et MOLLE [Biochem. J., 271, 247-252, (1990)] décrivent des peptides obtenus par hydrolyse tryptique du CMP ; toutefois, ils ne mentionnent pas leurs propriétés antigéniques.

La présente Invention s'est fixé pour but l'obtention de molécules antigéniques faciles à préparer par synthèse chimique, portant des déterminants antigéniques du CMP, et utilisables pour l'induction d'anticorps anti-CMP.

Les Inventeurs ont constaté que, bien que le CMP soit un glycopeptide, certains peptides non-glycosylés, représentant des fragments de la séquence du CMP, possédaient les propriétés souhaitées.

En outre, les Inventeurs ont constaté que parmi les peptides obtenus, certains ne présentaient pas ou peu de réactions croisées avec la caséine κ. Lorsque ces peptides sont utilisés comme immunogènes, ils permettent d'obtenir des anticorps spécifiques anti-CMP.

On entend par "anticorps spécifiques anti-CMP" des anticorps reconnaissant le CMP, et ne présentant pas de réactions croisées, ou bien uniquement des réactions faciles à éliminer par chromatographie d'affinité avec la caséine κ. L'obtention de tels anticorps à partir de fragments du CMP est surprenante étant donné l'impossibilité précédemment observée d'éliminer les réactions croisées avec la caséine κ lorsque le CMP est utilisé comme immunogène.

La présente Invention a pour objet l'utilisation d'un peptide antigénique choisi dans le groupe constitué par les peptides répondant à l'une des séquences suivantes :

Met-Ala-Ile-Pro-Pro ;
Met-Ala-Ile-Pro-Pro-Lys ;
Met-Ala-Ile-Pro-Pro-Lys-Lys ;
Met-Ala-Ile-Pro-Pro-Lys-Lys-Asn ;
Met-Ala-Ile-Pro-Pro-Lys-Lys-Asn-Gln ;
Met-Ala-Ile-Pro-Pro-Lys-Lys-Asn-Gln-Asp ;
Met-Ala-Ile-Pro-Pro-Lys-Lys-Asn-Gln-Asp-Lys ;
Ser-Pro-Pro-Glu-Ile-Asn-Thr-Val-Gln-Val ;

respectivement désignées dans la liste de séquences en annexe sous les numéros : SEQ. ID NO:1, SEQ. ID NO:11, SEQ. ID NO:10, SEQ. ID NO:9, SEQ. ID NO:8, SEQ. ID NO:7, SEQ. ID NO:6, SEQ. ID NO:2, pour l'obtention d'anticorps anti-CMP.

L'Invention a également pour objet un peptide antigénique répondant à l'une des séquences : SEQ. ID NO:1, SEQ. ID NO:11, SEQ. ID NO:10, SEQ. ID NO:9, SEQ. ID NO:8, SEQ. ID NO:7, SEQ. ID NO:6, SEQ. ID NO:2, et pourvu en outre d'une cystéine à son extrémité C-terminale.

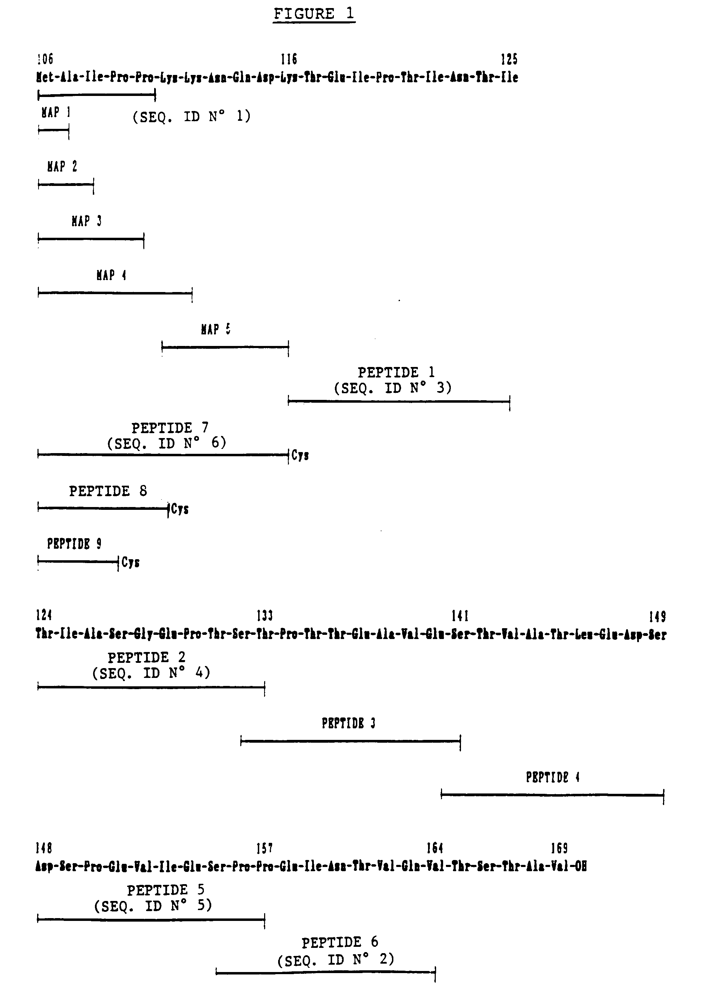

La position des peptides de séquences SEQ. ID N° 1, 2, 3, 4, 5, et 6 par rapport à la séquence du CMP est indiquée sur la figure 1, ainsi que celle des peptides utilisés pour la construction de MAP.

L'Invention englobe également des compositions antigéniques, lesquelles compositions sont caractérisées en ce qu'elles comprennent en tant que constituant essentiel au moins un peptide ou un dérivé de peptide choisi dans le groupe constitué par :
- tout peptide répondant à l'une des séquences : SEQ. ID NO:1, SEQ. ID NO:11, SEQ. ID NO:10, SEQ. ID NO:9, SEQ. ID NO:8, SEQ. ID NO:7, SEQ. ID NO:6, SEQ. ID NO:2, et pourvu en outre d'une cystéine à son extrémité C-terminale ;
- les produits de couplage d'un peptide répondant à l'une des séquences : SEQ. ID NO:1, SEQ. ID NO:11, SEQ. ID NO:10, SEQ. ID NO:9, SEQ. ID NO:8, SEQ. ID NO:7, SEQ. ID NO:6, SEQ. ID NO:2, éventuellement pourvu en outre d'une cystéine à son extrémité C-terminale, avec une protéine porteuse ;
- les MAP (multiple antigenic peptides) [TAM, Proc. Natl. Acad. Sci. USA, 85, 5409-5413, (1988)] obtenus à partir d'un peptide répondant à l'une des séquences : SEQ. ID NO:1, SEQ. ID NO:11, SEQ. ID NO:10, SEQ. ID NO:9, SEQ. ID NO:8, SEQ. ID NO:7, SEQ. ID NO:6, SEQ. ID NO:2, éventuellement pourvu en outre d'une cystéine à son extrémité C-terminale.

Ces compositions sont en particulier utilisables comme immunogènes pour l'induction d'anticorps anti-CMP, ainsi que comme réactifs pour la détection et le dosage du CMP.

Les compositions comprenant des peptides comportant la séquence SEQ. ID N° 1 (par exemple les compositions comprenant le peptide SEQ. ID N° 6 ou ses fragments) permettent l'obtention d'anticorps spécifiques anti-CMP.

Les compositions comprenant le peptide de séquence SEQ. ID N° 2 ou les fragments de celui-ci, permettent l'obtention d'anticorps spécifiques d'espèce, reconnaissant uniquement le CMP de lait de vache, ce qui permet la détection de la présence de lait de vache dans du lait ou des produits laitiers provenant prétendument d'autres espèces, en particulier dans du lait de brebis, de chèvre, et même dans du lait de bovins tels que le buffle

La présente Invention a également pour objet un procédé de préparation d'anticorps anti-CMP, lequel procédé est caractérisé en ce qu'il comprend une étape au cours de laquelle on immunise un animal avec une composition antigénique telle que définie ci-dessus, ou avec un peptide répondant à l'une des séquences : SEQ. ID NO:1, SEQ. ID NO:11, SEQ. ID NO:10, SEQ. ID NO:9, SEQ. ID NO:8, SEQ. ID NO:7, SEQ. ID NO:6, SEQ. ID NO:2.

L'Invention englobe également les anticorps anti-CMP susceptibles d'être obtenus par le procédé conforme à l'Invention.

Selon un mode de réalisation préféré de la présente Invention, lesdits anticorps sont des anticorps monoclonaux.

Selon un autre mode de réalisation préféré de la présente Invention, lesdits anticorps sont des anticorps polyclonaux.

La présente Invention a en outre pour objet un procédé d'évaluation de la protéolyse du lait et des produits laitiers, lequel procédé est caractérisé en ce qu'il comprend une étape au cours de laquelle on met en contact un anticorps spécifique anti-CMP conforme à l'Invention, produit contre un peptide répondant à l'une des séquences : SEQ. ID NO:1, SEQ. ID NO:11, SEQ. ID NO:10, SEQ. ID NO:9, SEQ. ID NO:8, SEQ. ID NO:7, ou SEQ. ID NO:6, avec un échantillon de lait ou d'un produit laitier à tester, et une étape où l'on procède à la mise en évidence et/ou au dosage par tous moyens appropriés, du complexe antigène/anticorps formé au cours de l'étape précédente.

Les anticorps anti-CMP qui reconnaissent à la fois la caséine κ et le CMP peuvent être utilisés à titre de contrôle pour évaluer la quantité totale de caséine κ (hydrolysée ou non-hydrolysée) présente.

Le procédé conforme à l'Invention a ainsi permis la mise en évidence par les Inventeurs de l'existence d'une protéolyse de la caséine κ dans les yaourts, protéolyse dont le degré varie selon les souches utilisées pour la fabrication du yaourt. Ce procédé peut donc avantageusement être appliqué pour la sélection des souches selon leur pouvoir de production du CMP.par hydrolyse de la caséine κ.

La présente Invention a en outre pour objet un procédé de détection de la présence de CMP de lait de vache dans le lait et les produits laitiers, lequel procédé est caractérisé en ce qu'il comprend une étape au cours de laquelle on met en contact un anticorps produit contre un peptide de séquence SEQ. ID N° 2 ou un fragment dudit peptide avec un échantillon de lait ou d'un produit laitier à tester, et une étape où l'on procède à la mise en évidence et/ou au dosage par tous moyens appropriés, du complexe antigène/anticorps formé au cours de l'étape précédente.

Les moyens de mise en évidence et de dosage d'un complexe antigène/anticorps sont bien connus de l'homme du métier ; on citera à titre d'exemple non limitatif, parmi les procédés les plus couramment employés, les différentes méthodes ELISA, les RIA, etc...

La présente Invention a également pour objet des réactifs de diagnostic pour le dosage du CMP, lesquels réactifs sont caractérisés en ce qu'ils comprennent au moins un peptide ou une composition antigénique ou bien au moins un anticorps anti-CMP conformes à l'Invention. Ledit anticorps ou ledit peptide sont éventuellement couplés à un marqueur approprié (chimique, enzymatique, ou radioactif) permettant la révélation du produit de la réaction.

La présente Invention sera mieux comprise à l'aide du complément de description qui va suivre, qui se réfère à des exemples de mise en oeuvre d'une composition antigénique conforme à l'Invention pour l'obtention d'anticorps anti-CMP et à l'utilisation desdits anticorps pour l'évaluation de la protéolyse du lait.

Il va de soi toutefois que ces exemples sont donnés uniquement à titre d'illustration de l'objet de l'Invention, dont ils ne constituent en aucune manière une limitation.

### I. - OBTENTION D'ANTICORPS ANTI-CMP

Les peptides sont synthétisés par la méthode de MERRIFIELD, en utilisant le protocole décrit dans le fascicule "Synthetic polypeptides as antigens", [VAN REGENMORTEL, BRIAND, MULLER, PLANE, Ed. Elsevier (1988)], puis purifiés par chromatographie. Après purification, le peptide est couplé à une protéine porteuse
- soit l'ovalbumine sur l'acide aminé Cystéine par l'agent couplant MBS (M-Maléimidobenzoyl-N-hydroxysuccinimide ester,
- soit la sérum albumine-bovine sur l'acide aminé tyrosine par l'agent couplant BDB (Bisdiazobenzidine).

### EXEMPLE 1 - Préparation d'un sérum polyclonal

Des lapins sont immunisés contre le peptide synthétique, selon le protocole suivant pour la première injection, 200 µg du peptide sont dissous dans 1 ml d'un mélange à volumes égaux de sérum physiologique et d'adjuvant complet de Freund. Des injections de rappel sont effectuées toutes les 3 semaines, avec 200 µg de peptide, en émulsion dans de l'adjuvant incomplet de Freund.

Les prélèvements de sang sont effectués à partir de 10 jours après la 1ère injection, et le sérum est recueilli.

Pour éliminer les réactions croisées avec la caséine κ, le sérum est adsorbé sur un gel de caséines κ, constitué de 10 mg de caséine κ, additionnés de 200 mg de sérum albumine bovine et d'un agent couplant le glutaraldéhyde à 2,5 %. Le gel polymérisé est conservé dans du tampon phosphate 0,1 M pH 7,2 à 4°C. Dans un premier temps, le gel est centrifugé 15 mn à 18600 g, à 4°C. Puis, il est centrifugé 15 mn à 23600 g, à 4°C pour éliminer le tampon phosphate.

Le sérum anti-CMP est ajouté sur le gel, à raison de 310 mg de sérum lyophilisé dans 1 ml de tampon phosphate. Ce mélange est agité 2 h à température ambiante, puis une nuit à 4°C. Ensuite, le gel est centrifugé 15 mn à 23600 g, à 4°C. Le surnageant est récupéré et le gel est rincé avec 0,25 ml de tampon phosphate. Puis, il est recentrifugé 15 mn à 23600 g afin de récupérer tout le sérum prisonnier. Le surnageant ainsi obtenu est additionné au premier surnageant.

### EXEMPLE 2 - Obtention d'anticorps monoclonaux anti-CMP

Des souris Balb/c BYJICO sont immunisées par une première injection intra-péritonéale de 100 µg de peptide couplé à l'ovalbumine, émulsifiés dans de l'adjuvant de Freund complet. Au 17ème jour, on procède à une injection intra-péritonéale de 50 µg de peptide couplé à l'ovalbumine, en présence d'adjuvant de Freund incomplet, et à une injection intra-veineuse de 10 µg de peptide couplé à l'ovalbumine, sans adjuvant. Au 20è jour, les souris sont sacrifiées, la rate est prélevée, et les splénocytes sont fusionnés à des cellules myélomateuses selon la méthode de MILSTEIN et KOHLER [Nature, 256, 495-497 (1975)]. Le criblage des hybrides obtenus est effectué sur le peptide ayant servi à l'immunisation couplé à la Sérum albumine Bovine.

### EXEMPLE 3 - Comparaison des propriétés antigéniques de différents peptides du CMP

Des peptides synthétiques constituant des fragments de la séquence du CMP de lait de vache, représentés à la figure 1, sous les numéros 1 à 9 (le peptide 7 correspond au peptide SEQ. ID N° 6 et le peptide 8 aux 6 premiers acides aminés de ladite séquence ; un résidu Cys a été ajouté à l'extrémité C-terminale de ces deux peptides ) ont été utilisés pour préparer des anticorps selon le procédé décrit dans l'Exemple 1.

Le dosage a été effectué par ELISA selon la procédure suivante 100 µl d'une solution de CMP à 2 µg/ml ou 100 µl d'une solution de caséine κ à 6 µg/ml sont déposés dans les puits de plaques de microtitration. On procède à l'adsorption de ces antigènes par incubation des plaques, une nuit à 4°C. Les cupules sont ensuite lavées trois fois avec 350 µl de PBS-Tween et sont saturées avec 250 µl de PBS-Tween + gélatine à 1%. La plaque est incubée 1h à 37°C. 100 µl de l'anticorps (dilution 1/1000e) produit contre un des peptides 1 à 8 sont déposés dans les cupules de chacune des plaques qui sont ensuites incubées 1 heure 30 à 37°C.

Après 3 lavages avec 350 µl de PBS-Tween, on additionne 100 µl par cupule d'un anticorps anti-IgG de lapin couplé à la peroxydase et dilué dans du PBS-Tween au 1/2000, et la plaque est incubée 45 mn à 37°C. Après 3 nouveaux lavages au PBS-Tween, on procède à la révélation. Le substrat utilisé est le 3,3',5,5' tétraméthylbenzidine (TMB), dilué au 1/10 dans du tampon citrate 0,1 M pH 5 + H₂O₂. 75 µl sont déposés dans chaque cupule, et la plaque est incubée 15 mn à 37°C.

La réaction entre la peroxydase et le TMB provoque une coloration bleue. Cette réaction est stoppée par l'ajout de 25 µl d'acide chlorhydrique 1N par cupule. Le substrat prend alors une coloration jaune. Les résultats sont évalués par mesure de la DO à 450 nm.

Les résultats sont représentés dans le tableau I ci-dessous (+) indique une réaction positive, (-) indique une réaction négative, le nombre de signes + indique l'intensité de la coloration observée

**TABLEAU I**

| ANTIGENE | | |
|---|---|---|
| ANTICORPS | CMP | caséine κ |
| 1 | +++ | +++ |
| 2 | ++ | ++ |
| 3 | - | - |
| 4 | - | - |
| 5 | +++ | +++ |
| 6 | +++ | +++ |
| 7 | +++ | + |
| 8 | +++ | - |
| 9 | - | - |

### II. - UTILISATION DES ANTICORPS ANTI-CMP POUR LE DOSAGE DU CMP DANS DES LAITS ET DES PRODUITS LAITIERS

Le dosage est effectué sur une solution de l'échantillon à tester ayant subi au préalable un traitement d'ultrafiltration, afin d'éliminer au maximum les protéines du lait qui, en s'adsorbant sur les cupules, interfereraient avec l'adsorption du CMP. Ce dosage est effectué par la méthode ELISA, comme décrit dans l'exemple 3 ci-dessus.

Une quantité connue d'antigène purifié (par exemple 100 µl d'une solution de CMP à 2 µg/ml ; les peptides ou les MAP conformes à l'Invention, peuvent également être utilisés en remplacement du CMP) est adsorbée sur la plaque.

Si l'on souhaite procéder au dosage sur un échantillon non ultrafiltré, il est préférable de procéder à un dosage ELISA de type compétitif.

Après lavage et saturation de la plaque, la réaction antigène-anticorps est effectuée en présence de dilutions successives de la solution antigénique à doser.

Le produit de la réaction est revélé comme indiqué à l'exemple 3 ci-dessus.

La quantité de CMP présente dans l'échantillon à doser est évaluée par comparaison avec une gamme étalon obtenue à partir de dilutions connues de CMP.

### EXEMPLE 4 - Dosage du CMP dans le lait

Les anticorps utilisés pour ce dosage ont été produits contre le peptide 7 (SEQ. ID N° 6 + Cys).

Une étude sur 110 échantillons de lait, provenant de 4 fromageries, a montré que 13 % de ces échantillons renfermant en moyenne 2.10⁵ germes par millilitre à leur arrivée au laboratoire, contenaient plus de 5 µg de CMP par millilitre. Après deux jours de conservation à +4°C, la moyenne des germes s'élevait à 4.10⁶ germes/ml et 26 % des échantillons possédaient plus de 5 µg de CMP par millilitre.

En outre, 9 échantillons de lait UHT ont été examinés, avant et après centrifugation, et leur déstabilisation a été évaluée. Les échantillons qui restaient stables après centrifugation ont été chauffés, entre 60 et 100°C et leur température de déstabilisation a été notée.

On observe que les laits très déstabilisés ont une forte concentration en CMP (>80 µg/ml) alors que les lait stables contiennent 10 µg de CMP par ml, voire moins. Un lait qui reste stable après centrifugation mais qui se déstabilise lors du chauffage contient 43,4 µg de CMP par ml. Les résultats font apparaître une relation entre la protéolyse de la caséine κ évaluée par dosage du CMP, et la déstabilisation des laits UHT.

### EXEMPLE 5 - Utilisation d'anticorps conformes à l'Invention pour le dosage du CMP dans le fromage et démonstration de la spécificité d'espèce d'anticorps dirigés contre le peptide SEQ ID N°2

Un anticorps polyclonal de lapin dirigé contre le peptide SEQ ID N° 2 et préparé selon le protocole décrit à l'Exemple 1 est utilisé.

Une extraction aqueuse dans 10 ml de tampon phosphate 0,05 M, pH 7,2 est effectuée sur 5 g de fromage par malaxage à l'aide d'un batteur. La présence ou l'absence de CMP de vache est recherchée sur la phase aqueuse obtenue par centrifugation de la solution obtenue après le malaxage en utilisant la méthode ELISA d'inhibition décrite précédemment.

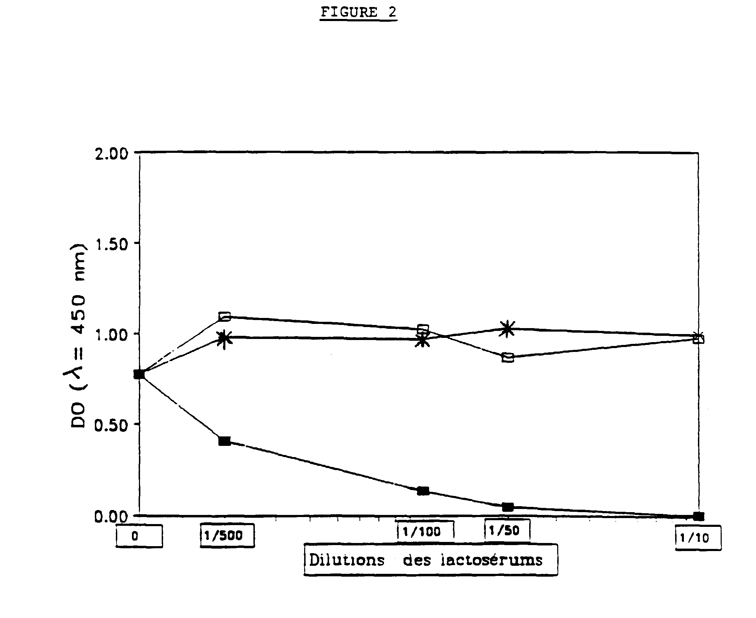

Les résultats sont illustrés par la figure 2. En abscisse sont indiquées les dilutions des extraits aqueux (■) vache ; ( 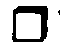 ) brebis ; ( 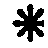 ) chèvre ; en ordonnée la DO 450.

### LISTE DES SEQUENCES

### SEQ ID N° 1

### Peptide

Acides aminés 106 à 110 de la caséine κ de lait de vache

Met-Ala-Ile-Pro-Pro

### SEQ ID N° 2

### Peptide

Acides aminés 155 à 164 de lait de vache

Ser-Pro-Pro-Glu-Ile-Asn-Thr-Val-Gln-Val

### SEQ ID N° 3

### Peptide

Acides aminés 116 à 125 de lait de vache

Lys-Thr-Glu-Ile-Pro-Thr-Ile-Asn-Thr-Ile,

### SEQ ID N° 4

### Peptide

Acides aminés 124 à 133 de lait de vache

Thr-Ile-Ala-Ser-Gly-Glu-Pro-Thr-Ser-Thr,

### SEQ ID N° 5

### Peptide

Acides aminés 148 à 157 de lait de vache

Asp-Ser-Pro-Glu-Val-Ile-Glu-Ser-Pro-Pro

### SEQ ID N° 6

### Peptide

Acides aminés 106 à 116 de lait de vache

Met-Ala-Ile-Pro-Pro-Lys-Lys-Asn-Gln-Asp-Lys

## Revendications

1. Utilisation d'un peptide antigénique choisi dans le groupe constitué par les peptides répoondant à l'une des séquences suivantes : 
Met-Ala-Ile-Pro-Pro
Met-Ala-Ile-Pro-Pro-Lys ;
Met-Ala-Ile-Pro-Pro-Lys-Lys ;
Met-Ala-Ile-Pro-Pro-Lys-Lys-Asn ;
Met-Ala-Ile-Pro-Pro-Lys-Lys-Asn-Gln ;
Met-Ala-Ile-Pro-Pro-Lys-Lys-Asn-Gln-Asp ;
Met-Ala-Ile-Pro-Pro-Lys-Lys-Asn-Gln-Asp-Lys ;
Ser-Pro-Pro-Glu-Ile-Asn-Thr-Val-Gln-Val ;
 respectivement désignées dans la liste de séquences en annexe sous les numéros : SEQ. ID NO:1, SEQ. ID NO:11, SEQ. ID NO:10, SEQ. ID NO:9, SEQ. ID NO:8, SEQ. ID NO:7, SEQ. ID NO:6, SEQ. ID NO:2, pour l'obtention d'anticorps anti-CMP.

2. Peptide antigénique caractérisé en ce qu'il est constitué par un peptide tel que défini dans la revendication 1, pourvu d'une cystéine à son extrémité C-terminale.

3. Composition antigénique caractérisée en ce qu'elle comprend en tant que constituant essentiel au moins un peptide ou un dérivé de peptide choisi dans le groupe constitué par :
- les peptides selon la revendication 2 ;
- les produits de couplage d'un peptide tel que défini dans une quelconque des revendications 1 ou 2, avec une protéine porteuse.
- les MAP dérivés d'un peptide tel que défini dans une quelconque des revendications 1 ou 2.

4. Procédé de préparation d'anticorps anti-CMP, lequel procédé est caractérisé en ce qu'il comprend une étape au cours de laquelle on immunise un animal avec une composition antigénique selon la revendication 3, ou avec un peptide tel que défini dans la revendication.

5. Anticorps anti-CMP caractérisés en ce qu'ils sont susceptibles d'être obtenus par le procédé selon la Revendication 4.

6. Anticorps selon la Revendication 5, caractérisés en ce qu'ils sont des anticorps monoclonaux.

7. Anticorps selon la Revendication 5, caractérisés en ce qu'ils sont des anticorps polyclonaux.

8. Procédé permettant l'évaluation de la protéolyse du lait et des produits laitiers, lequel procédé est caractérisé en ce qu'il comprend au moins une étape au cours de laquelle on met en contact un anticorps selon la revendication 5, produit contre un peptide repondant à l'une des séquences : SEQ. ID NO:1, SEQ. ID NO:11, SEQ. ID NO:10, SEQ. ID NO:9, SEQ. ID NO:8, SEQ. ID NO:7, ou SEQ. ID NO:6, avec un échantillon de lait ou de produit laitier à tester, et une étape où l'on procède à la mise en évidence et/ou au dosage par tous moyens appropriés, du complexe antigène/anticorps formé au cours de l'étape précédente.

9. Procédé de détection de la présence de CMP du lait de vache dans le lait et les produits laitiers, lequel procédé est caractérisé en ce qu'il comprend une étape au cours de laquelle on met en contact un anticorps selon la revendication 5, produit contre un peptide de séquence SEQ. ID NO:2, avec un échantillon de lait ou d'un produit laitier à tester, et une étape où l'on procède à la mise en évidence et/ou au dosage par tous moyens appropriés, du complexe antigène/anticorps formé au cours de l'étape précédente.

10. Réactifs pour la détection ou le dosage du CMP lesquels réactifs sont caractérisés en ce qu'ils comprennent au moins un anticorps anti-CMP, selon l'une quelconque des Revendications 4 à 6.

## Patentansprüche

1. Verwendung eines antigenen Peptids, ausgewählt aus der Gruppe, bestehend aus den Peptiden mit einer der nachstehenden Sequenzen 
Met-Ala-Ile-Pro-Pro
Met-Ala-Ile-Pro-Pro-Lys ;
Met-Ala-Ile-Pro-Pro-Lys-Lys ;
Met-Ala-Ile-Pro-Pro-Lys-Lys-Asn ;
Met-Ala-Ile-Pro-Pro-Lys-Lys-Asn-Gln ;
Met-Ala-Ile-Pro-Pro-Lys-Lys-Asn-Gln-Asp ;
Met-Ala-Ile-Pro-Pro-Lys-Lys-Asn-Gln-Asp-Lys ;
Ser-Pro-Pro-Glu-Ile-Asn-Thr-Val-Gln-Val ;
 die in dem als Anhang beigefügten Sequenzprotokoll jeweils mit den Nummern SEQ. ID NO:1, SEQ. ID NO:11, SEQ. ID NO:10, SEQ. ID NO 9, SEQ. ID NO:8, SEQ. ID NO:7, SEQ. ID NO:6, SEQ. ID NO:2 bezeichnet sind, zum Erhalt von anti-CMP Antikörpern.

2. Antigenes Peptid, dadurch **gekennzeichnet,** daß es aus einem Peptid nach Anspruch 1, versehen mit einem Cystein an seinem C-terminalen Ende, besteht.

3. Antigene Zusammensetzung, dadurch **gekennzeichnet,** daß sie als wesentlichen Bestandteil mindestens ein Peptid oder ein Derivat eines Peptids, ausgewählt aus der Gruppe, bestehend aus
- den Peptiden nach Anspruch 2,
- den Kopplungsprodukten eines Peptids nach einem der Ansprüche 1 oder 2 mit einem Trägerprotein
- den MAP-Derivaten eines Peptids nach einem der Ansprüche 1 oder 2
umfaßt.

4. Verfahren zur Produktion von anti-CMP Antikörpern, dadurch **gekennzeichnet,** daß es eine Stufe umfaßt, bei der man ein Tier mit einer antigenen Zusammensetzung nach Anspruch 3 oder mit einem Peptid wie in dem Anspruch definiert, immunisiert.

5. Anti-CMP Antikörper, dadurch **gekennzeichnet,** daß sie durch das Verfahren nach Anspruch 4 erhalten werden können.

6. Antikörper nach Anspruch 5, dadurch **gekennzeichnet**, daß sie monoclonale Antikörper sind.

7. Antikörper nach Anspruch 5, dadurch **gekennzeichnet,** daß sie polyclonale Antikörper sind.

8. Verfahren zur Bewertung der Proteolyse von Milch und Milchprodukten, dadurch **gekennzeichnet,** daß es mindestens eine Stufe umfaßt, in deren Verlauf man einen Antikörper nach Anspruch 5, der gegen ein Peptid mit einer der Sequenzen SEQ. ID NO:1, SEQ. ID NO:11, SEQ. ID NO:10, SEQ. ID NO:9, SEQ. ID NO:8, SEQ. ID NO:7, SEQ. ID NO:6 erhalten ist mit einer zu testenden Probe der Milch oder des Milchprodukts in Kontakt bringt und eine Stufe, bei der man den im Verlauf der vorstehenden Stufe gebildeten Antigen/Antikörperkomplex durch jedes geeignete Mittel nachweist und/oder bestimmt.

9. Verfahren zum Nachweis der Anwesenheit von CMP der Kuhmilch in Milch und Milchprodukten, dadurch **gekennzeichnet,** daß es mindestens eine Stufe, im Verlauf derer man einen Antikörper nach Anspruch 5, der gegen ein Peptid der Sequenz SEQ. ID NO:2 erhalten ist mit der zu testenden Probe der Milch oder eines Milchprodukts in Kontakt bringt, und eine Stufe, bei der man den im Verlauf der vorstehenden Stufe gebildeten Antigen/Antikörperkomplex mit jedem geeigneten Mittel nachweist und/oder bestimmt, umfaßt.

10. Reagentien zum Nachweis oder zur Bestimmung von CMP, dadurch **gekennzeichnet,** daß sie mindestens einen anti-CMP Antikörper nach einem der Ansprüche 4 bis 6 umfassen.

## Claims

1. Use of an antigenic peptide chosen from the group constituted by the peptides which satisfy one of the following sequences: 
Met-Ala-Ile-Pro-Pro
Met-Ala-Ile-Pro-Pro-Lys
Met-Ala-Ile-Pro-Pro-Lys-Lys
Met-Ala-Ile-Pro-Pro-Lys-Lys-Asn
Met-Ala-Ile-Pro-Pro-Lys-Lys-Asn-Gln
Met-Ala-Ile-Pro-Pro-Lys-Lys-Asn-Gln-Asp
Met-Ala-Ile-Pro-Pro-Lys-Lys-Asn-Gln-Asp-Lys
Ser-Pro-Pro-Glu-Ile-Asn-Thr-Val-Gln-Val
 respectively designated in the appended list of sequences under numbers: SEQ. ID NO:1, SEQ.ID NO:11, SEQ. ID NO:10, SEQ. ID NO:9, SEQ. ID NO:8, SEQ. ID NO:7, SEQ. ID NO:6, SEQ. ID NO:2, for obtaining anti-CMP antibodies.

2. An antigenic peptide characterised in that it consists of a peptide as defined in claim 1 provided with a cystine at its C-terminal end.

3. An antigenic composition characterised in that it comprises as an essential constituent at least one peptide or peptide derivative chosen from the group constituted by:
- the peptides according to claim 2,
- the products of coupling a peptide as defined in either of Claims 1 or 2 with a carrier protein,
- the MAPs derived from a peptide as defined in either of claims 1 or 2.

4. A method of preparing anti-CMP antibodies, the said method being characterised in that it comprises a stage during the course of which an animal is immunised with an antigenic composition or with a peptide according to Claim 3.

5. Anti-CMP antibodies, characterised in that they are capable of being obtained by the method according to claim 4.

6. Antibodies according to claim 5, characterised in that they are monoclonal antibodies.

7. Antibodies according to claim 5, characterised in that they are polyclonal antibodies.

8. A method permitting evaluation of the proteolysis of milk and dairy products, the said method being characterised in that it comprises at least one stage during the course of which an antibody according to claim 5, produced against a peptide satisfying one ofthe sequences: SEQ. ID NO:1, SEQ. ID NO:11, SEQ. ID NO:10, SEQ. ID NO:9, SEQ. ID NO:8, SEQ. ID NO:7 or SEQ. ID NO:6, is brought into contact with a sample of milk or dairy product to be tested and a stage in which the antigenic complex/antibody formed during the course of the preceding stage is revealed and/or quantitively measured by any appropriate means.

9. A method of detecting the presence of CMP in cow's milk in milk and dairy products, the said method being characterised in that it comprises a stage during the course of which an antibody according to claim 5 produced against the peptide of sequence SEQ. ID NO:2 is brought into contact with a sample of milk or dairy product to be tested, and a stage in which the antigenic complex/antibody formed during the course of the preceding stage is revealed and/or quantitively measured by any appropriate means.

10. Reagents for detecting or quantitively measuring CMP, the said reagents being characterised in that they comprise at least one anti-CMP antibody according to any one of claims 4 to 6.
